# EUROPEAN PATENT SPECIFICATION

(11) **EP 0 936 880 B1**
(45) Date of publication and mention of the grant of the patent: **20.04.2005**
(21) Application number: 97946353.6
(22) Date of filing: 29.10.1997
(51) Int. Cl.: A41B 9/12, A41D 13/00

(54) **PROTECTIVE GARMENT FOR THE HIP AREA**
BEKLEIDUNG MIT HÜFTGELENKSCHUTZ
VETEMENT DE PROTECTION DE LA REGION DE LA HANCHE

(30) Priority: 29.10.1996 US 744713
(43) Date of publication of application: 25.08.1999
(73) Proprietor: Plum Enterprises, Inc., Valley Forge, PA 19481-0085 (US)
(72) Inventor: CARRINGTON, Janice, Valley Forge, PA 19481-0085 (US)
(74) Representative: Bradley, Josephine Mary
(86) International application number: PCT/US1997/019603
(87) International publication number: WO 1998/018355

(56) References cited:
- WO-A-95/19154
- WO-A-96/20615
- DE-A- 3 638 718
- US-A- 3 068 871
- US-A- 3 945 042
- US-A- 4 561 123
- US-A- 4 807 301
- US-A- 4 987 613
- US-A- 5 052 052
- US-A- 5 105 473
- US-A- 5 365 610
- US-A- 5 461 730
- US-A- 5 497 511
- US-A- 5 551 082
- US-A- 5 592 689
- US-A- 5 636 377

## Description

### Background Of The Invention

The invention relates to a shock and stress protective garment for the hip area, and more particularly, to a protective garment that can be worn comfortably and can disperse forces directed toward the hip area, absorbing such forces in the vulnerable regions of the hip.

There are over 300,000 hip fractures each year in the United States. Additionally, there are many other types of hip joint injuries that result in pain. Complications associated with hip injuries, such as pneumonia, can result in disruption of normal life, substantial medical costs and even death. These fractures and other injuries are particularly common among the elderly, who experience degenerative changes in bone and tissue structure with advancing age. The degenerative changes become much worse after a hip fracture. In view of ever increasing life expectancies, the number of injuries of this type and the costs associated with them can be expected to increase with time.

The hip joint is an enarthrodial or ball-and-socket joint formed by the reception of the ball-shaped head on the upper or proximal end of the femur into the cup-shaped cavity in the pelvis called the acetabulum. A fall or blow to the hip bone area, if the area is unprotected, can result in body tissue injuries, dislocation of the femur head from the acetabulum, and fractures of the acetabulum or various parts of the proximal femur or other damage in the vicinity of the hip. Particularly vulnerable is the so-called greater trochanter which protrudes outwardly from the proximal femur just below the joint and the adjoining thin neck of the femur. This region is relatively poorly protected by muscle and other body tissue in comparison with the regions of the hip surrounding it. In fact, the greater trochanter is readily accessible to the touch, its position being generally indicated by an elevation in the hip area due to the thinness of the tissues that cover it.

Prior art garments that are capable of providing a measure of protection with respect to these problems are usually difficult to apply and uncomfortable to wear. An effective hip area shock and stress protective garment that is sufficiently comfortable to wear for extended periods of time under clothing, including during normal daytime activities, as well as while sleeping at night, is not available.

In the prior art, U.S. Patent No. 4,641,641 discloses an annular pad of resilient material that carries adhesive strips on one surface to secure a pad directly to the skin and a dome-shaped shield. U.S. Patent No. 4,573,216 discloses a pad that protects only small isolated areas, such as the immediate area where the greater trochanter approaches the surface of the body. It depends on adhesive to fix it to the skin. The adhesive taught in this manner is not comfortable for long periods of wear. U.S. Patent No. 2,889,830 is designed to protect only the area of the greater trochanter. Furthermore, it is bulky in design and uncomfortable because of its hard component parts and straps. The bulky components make it undesirable from a cosmetic perspective. The same is true of U.S. Patent No. 3,526,221. The protective device taught by U.S. Patent No. 3,526,221 protects only the area of the greater trochanter and is designed in such a way that it is uncomfortable to wear for extended periods of time. U.S. Patent Nos. 1,756,358 and 1,774,739 are hard, uncomfortable devices worn under clothing designed primarily for sports. These devices are also undesirable from a cosmetic perspective.

WO 95/19154 (Andresen) provides a hip protector consisting of a domed shell of rigid polypropylene foam material, optionally with a rigid or semi-rigid polypropylene core. The protector is 13 to 18 cm high and 9 to 14 cm wide. WO 96/20615 (Kristensen) describes a very similar product.

U.S. Pat. No. 3,945,042 (Lobo) describes a jacket having a cushioning structure consisting of an array of cylindrical ribs of foam material sewn into the lower part around the rear three quarters of the hip area. The bulk density of the material is up to 240 kg/m³.

U.S. Pat. No. 4,807,301 (Ferber), on which the preamble of claim 1 is based, discloses a protective girdle with closed-cell foam pads extending around about two thirds of the circumference of the hips of the wearer. There is a slit or "dart" at the top center. The pads may be perforated for ventilation.

U.S. Pat. No. 5,636,377 (Wiener) provides a pad formed of one or two layers of foam material. The pad is 175 mm high and in the largest size is 125 mm wide.

Thus, the prior art patents teach many devices for the protection of the hip area. However, the devices taught in the prior art do not solve the problem of providing an effective, light, comfortable hip protective garment which is cosmetically acceptable.

### Summary

Accordingly, a primary object of the present invention is to provide a shock and stress protective garment that reduces the likelihood of fracture or other injury of the hip or surrounding area and is cosmetically acceptable.

Another object of the invention is to provide a shock and stress protective garment that can be worn and removed easily without straps, bandages or other devices that are difficult to apply and manipulate, especially by older people with arthritic or weak hands.

Another object of the invention is to provide a shock and stress protective garment for the hip area that is lightweight and comfortable to wear for extended periods of time.

Another object of the invention is to provide a shock and stress protective garment for the hip area that is relatively unobtrusive physically and cosmetically, and is comfortable to wear.

The invention provides a protective garment for protecting the enarthrodial joint of a hip of a wearer against shock, comprising: a protective area comprising a shock absorbing pad; and a non-protective area; the protective pad being sized and positioned to cover the front, back, and side of the enarthrodial joint when the garment is worn by the wearer; characterized in that the protective pad is provided with a plurality of intersecting slits.

### Detailed Description of the Invention

The protective garment of the present invention is a girdle-like garment that can be worn by a user to absorb shocks and stresses to the hip regions of the body and thereby prevent hip fractures and other types of hip injuries that can result from such shocks and stresses. For example, the protective garment can be used to prevent hip injuries due to shocks and stresses to the hip regions associated with falls suffered by the user.

The protective garment includes protective areas and nonprotective areas. Protective areas of the protective garment are disposed adjacent the hip regions of the body of the user for absorbing shocks applied to the hip regions and thereby protecting the hip regions from injury. Nonprotective areas of the protective garment can be formed of conventional materials that are associated with conventional girdles for providing structural integrity to the protective garment, while maintaining protective areas in their desired locations adjacent the vicinity of the hip regions of the user. Nonprotective areas of the protective garment can extend from the waist of the user at an elastic band to or below the thigh region.

Protective regions are disposed adjacent the side of the body of the user of the protective garment such that protective regions are approximately centered over an area of the user's femur that is slightly below the neck of the femur. Thus protective regions are approximately centered slightly below the acetabulum of the pelvis on the sides of the user. Protective regions extend from the sides of the user anteriorly and posteriorly around the body of the user in order to partially encircle and protect the regions in the vicinity of the ball-and-socket hip joint.

As a protective region extends anteriorly around the body of the user it also extends upwardly so that it passes over the ball-and-socket joint where the proximal head of the femur is received by the acetabulum of the pelvis. In this manner the protective region is approximately centered over the acetabulum on the front of the user. Preferably, the protective region extends upwardly to a point at least slightly beyond the curvature of the pelvis.

As the protective region extends posteriorly from the side of the user, it also extends downwardly. In this manner it is formed to pass along the lower region of the gluteus muscles of the user. In the preferred embodiment of the invention, the protective region is formed to extend about one-half of the way across the lower region of the buttock of the user.

The size of the protective regions varies as needed in order to fit individual users in the manner shown and set forth herein. Thus, there can be a wide range of possible sizes of protective regions. In a prototype model of the protective garment a protective region of a size acceptable for an individual of normal size had a dimension of approximately 23 cm. at its maximum length and a dimension of approximately 13 cm. at its maximum width. Although this size was believed to be suitable for the prototype, it will be understood that substantially different sizes are required depending on the size of the user. Thus, the size is selected as required to cover the necessary region as previously specified according to the individual user.

It will be understood that protective regions can be formed substantially larger than as set forth herein with respect to the hip area. However, in order to provide the most comfort for extended periods of wear and to provide the least cosmetic intrusiveness, it is preferred that the protective regions be the minimum size consistent with good protection of the user from hip injuries.

The protective areas include shock absorbing pads that are formed of a shock absorbing core and shell pieces for encapsulating the shock absorbing core. The shock absorbing core, and hence the shell pieces with which it is associated, is shaped to be operatively disposed so that it overlies and fully protects the hip of the user as previously described.

In the preferred embodiment of the invention, when the shock absorbing core is enclosed by the shell pieces, the shell pieces are stitched together around their peripheries and outside the periphery of the core. Thus, the size and shape of the shell pieces should approximate the size and shape of the core with which they are associated. Details of the construction and materials of pads suitable for use as these shock absorbing pads are set forth in detail in U.S. Patent No. 5,461,730.

The shock absorbing pads can be either removably attached to nonprotective areas or fixed to nonprotective areas in order to form protective regions. The core of the pads is preferably formed of a high density closed cell impact absorbent material such as Ensolite HH. It will be understood that use of a high density foam rather than a relatively lower density foam permits the core to be made thinner and more cosmetically acceptable for a desired amount of shock absorption. This is desirable since the protective garment is adapted to be worn as an undergarment and it is preferable that it not be detectable. Although closed cell foam is preferred for forming the core, it is understood that open cell foam can be used in keeping with the spirit of the invention if closed cell foam is desired for a particular reason. Additionally, the core can be formed of sponge material. It will also be understood that the shock absorbent core can be made of any number of layers of foam wherein the foam layers forming the core can have different densities. The shell pieces for encapsulating the core can be formed of a cotton knit stretch interlock fabric.

The shock absorbing core is provided with a plurality of intersecting slits . Slits facilitate the conforming of the initially flat core to the compound curvatures of the hip region of the user of the protective garment. Another advantage provided by the slits of the core is that slits enhance ventilation thereby making the protective garment more comfortable during extended periods of wear by the user.

The material from which the shell pieces are formed can be any suitable fabric. For example, the preferred materials used to form the shell pieces can include cotton, nylon, lycra, polyester, spandex, elastic and lace. Such materials provide a desirable degree of durability and soil resistance, as well as an acceptable feel and conventional appearance to the protective garment.

Shock absorbing cores can be formed with a thickness between 0,3175 cm (1/8 inch) and 2,54 cm (1 inch) if sufficiently dense foam is used. Thicker cores can be beveled for a smoother appearance under the clothing of the user. The cores can be die cut to fit the contour of the hip region as described hereinabove. Additionally, the shock absorbing cores can be provided with curvatures in order to prevent the pads from restricting the leg movement of the user.

Shock absorbing pads can be sewn into garments or detachably adhered to garments, such as a suitably modified conventional girdle, in order to form the protective garment of the present invention. For example, shock absorbing pads can be adhered to such a garment using Velcro. Furthermore, the positioning of the pads can be customized to the physique of an individual user and to the individual protection requirements of a user using this method of attaching pads. For example, multiple pieces of Velcro can be sewn inside the garment in order to permit selective positioning of shock absorbing pads for this purpose.

It will be understood by those skilled in the art that because the shock absorbing pads are formed of closed cell foam, they do not absorb water, and that they are therefore substantially waterproof. Thus, it is not necessary to remove them from the protective garment when laundering the protective garment. Additionally, the protective garment including the pads can, for this reason, be worn under swim wear.

The protective garment can be provided with abdominal support for supporting the abdomen of the user and urging the abdomen of the user inwardly. The abdominal support of the protective garment can be formed of a nonwoven interfacing or additional stretch lycra down the center front of the protective garment or any other method for providing an inward bias. The protective garment can also be provided with an uplift (not shown) for supporting the gluteus muscles of the user. Additionally, a hidden stretch lock pouch (not shown)can be provided within the protective garment in order to hold incontinence pads if needed by the user. The protective garment can also be formed as an all-in-one undergarment (not shown) including a brassier as well as a girdle.

The protective garment can also be provided with lower back support. The lower back support of the protective garment is effective to stabilize the sacral iliac spine of the user. It can be formed, for example, by providing plastic stays sewn,into the protective garment , by adding further layers of lycra to the material forming the sacral iliac spine region of the protective garment, or by providing a combination of nylon and neoprene to the region. Any of these methods are effective to provide a small area concealed brace to form a back support within the protective garment .

The present invention may be embodied in other specific forms without departing from the scope of the appended claims.

## Claims

1. A protective garment for protecting the enarthrodial joint of a hip of a wearer against shock, comprising:
a protective area comprising a shock absorbing pad;
a non-protective area;
the protective pad being sized and positioned to cover the front, back, and side of the enarthrodial joint when the garment is worn by the wearer;
**characterized in that** the protective pad is provided with a plurality of intersecting slits.

2. The protective garment of claim 1, wherein said protective pad is resilient.

3. The protective garment of claim 1, wherein said protective pad comprises foam material.

4. The protective garment of claim 1, wherein said protective pad is beveled.

5. The protective garment of claim 3, wherein said foam material comprises closed cell foam material.

6. The protective garment of claim 3, wherein said foam material comprises open cell foam material.

7. The protective pad garment of claim 1, wherein said protective pad comprises a pad core and a pad shell.

8. The protective garment of claim 7, wherein said pad core comprises more than one layer.

9. The protective garment of claim 8, wherein said layers comprise layers of different densities.

10. The protective garment of claim 7, wherein said pad shell encloses said pad core.

11. The protective garment of claim 7, wherein said pad shell is formed of cotton.

12. The protective garment of claim 7, wherein said pad shell comprises a cotton knit interfacing fabric.

13. The protective garment of claim 1, wherein said protective pad has a thickness of between 3 and 25mm.

14. The protective garment of claim 1, wherein said nonprotective area is formed of fabric.

15. The protective garment of claim 14, wherein said fabric comprises cotton.

16. The protective garment of claim 14, wherein the material forming said fabric is selected from the group consisting of nylon, lycra, polyester and spandex.

17. The protective garment of claim 1, wherein said protective pad is detachably secured to said nonprotective area.

18. The protective garment of claim 1, wherein said protective pad is fixed to said nonprotective area.

19. The protective garment of Claim 1, comprising a plurality of protective pads.

20. The protective garment of Claim 17, wherein each protective pad of said plurality of protective pads is disposed adjacent an enarthroidal joint region.

21. The protective garment of Claim 1, wherein said protective garment comprises a panty girdle.

22. The protective garment of Claim 1, further comprising an abdomen support.

23. The protective garment of Claim 22, wherein said abdomen support is disposed on the center of the front of said protective garment.

24. The protective garment of Claim 22, wherein said abdomen support comprises a non-woven interfacing.

25. The protective garment of Claim 22, wherein said abdominal support comprises a stretch material.

26. The protective garment of Claim 1, further comprising a gluteal support.

27. The protective garment of Claim 1, further comprising a back support panel.

28. The protective garment of Claim 1, wherein said protective area is approximately centered below the level of said acetabulum of said pelvis on said side of said body.

29. The protective garment of claim 1 or claim 28, wherein said protective area is approximately centered over said acetabulum of said pelvis on the front of said body.

30. The protective garment of Claim 1, further comprising means for attaching said protective pad to one of a plurality of different locations within said protective garment.

## Patentansprüche

1. Eine schützende Bekleidung zum Schützen des Nussgelenks einer Hüfte eines Trägers gegen Stöße, die Folgendes beinhaltet:
einen schützenden Bereich, der ein Stoßdämpfpolster beinhaltet;
einen nicht schützenden Bereich;
wobei das schützende Polster so bemessen und positioniert ist, dass es die Vorderseite, Rückseite und Seite des Nussgelenks bedeckt, wenn die Bekleidung von dem Träger getragen wird;
**dadurch gekennzeichnet, dass** das schützende Polster mit einer Vielzahl von sich schneidenden Schlitzen versehen ist.

2. Schützende Bekleidung gemäß Anspruch 1, wobei das schützende Polster elastisch ist.

3. Schützende Bekleidung gemäß Anspruch 1, wobei das schützende Polster Schaumstoffmaterial beinhaltet.

4. Schützende Bekleidung gemäß Anspruch 1, wobei das schützende Polster abgeschrägt ist.

5. Schützende Bekleidung gemäß Anspruch 3, wobei das Schaumstoffmaterial Schaumstoffmaterial mit geschlossenen Poren beinhaltet.

6. Schützende Bekleidung gemäß Anspruch 3, wobei das Schaumstoffmaterial offenporiges Schaumstoffmaterial beinhaltet.

7. Schützende Bekleidung mit Polster gemäß Anspruch 1, wobei das schützende Polster einen Polsterkern und einen Polstermantel beinhaltet.

8. Schützende Bekleidung gemäß Anspruch 7, wobei der Polsterkern mehr als eine Schicht beinhaltet.

9. Schützende Bekleidung gemäß Anspruch 8, wobei die Schichten Schichten unterschiedlicher Dichten beinhalten.

10. Schützende Bekleidung gemäß Anspruch 7, wobei der Polstermantel den Polsterkern umhüllt.

11. Schützende Bekleidung gemäß Anspruch 7, wobei der Polstermantel aus Baumwolle gebildet ist.

12. Schützende Bekleidung gemäß Anspruch 7, wobei der Polstermantel ein aus Baumwolle gewirktes Einlagegewebe beinhaltet.

13. Schützende Bekleidung gemäß Anspruch 1, wobei das schützende Polster eine Dicke von zwischen 3 und 25 mm aufweist.

14. Schützende Bekleidung gemäß Anspruch 1, wobei der nicht schützende Bereich aus einem Gewebe gebildet ist.

15. Schützende Bekleidung gemäß Anspruch 14, wobei das Gewebe Baumwolle beinhaltet.

16. Schützende Bekleidung gemäß Anspruch 14, wobei das das Gewebe bildende Material aus der Gruppe, bestehend aus Nylon, Lycra, Polyester und Elasthan, ausgewählt ist.

17. Schützende Bekleidung gemäß Anspruch 1, wobei das schützende Polster an dem nicht schützenden Bereich abnehmbar gesichert ist.

18. Schützende Bekleidung gemäß Anspruch 1, wobei das schützende Polster an dem nicht schützenden Bereich fixiert ist.

19. Schützende Bekleidung gemäß Anspruch 1, die eine Vielzahl von schützenden Polstern beinhaltet.

20. Schützende Bekleidung gemäß Anspruch 17, wobei jedes schützende Polster der Vielzahl von schützenden Polstern anliegend an eine Nussgelenk-Region angeordnet ist.

21. Schützende Bekleidung gemäß Anspruch 1, wobei die schützende Bekleidung eine Miederhose beinhaltet.

22. Schützende Bekleidung gemäß Anspruch 1, die ferner eine Bauch-Stütze beinhaltet.

23. Schützende Bekleidung gemäß Anspruch 22, wobei die Bauch-Stütze auf der Mitte der Vorderseite der schützenden Bekleidung angeordnet ist.

24. Schützende Bekleidung gemäß Anspruch 22, wobei die Bauch-Stütze eine nicht gewebte Einlage beinhaltet.

25. Schützende Bekleidung gemäß Anspruch 22, wobei die Bauch-Stütze ein dehnbares Material beinhaltet.

26. Schützende Bekleidung gemäß Anspruch 1, die ferner eine Gesäßstütze beinhaltet.

27. Schützende Bekleidung gemäß Anspruch 1, die ferner eine Rücken-Stützplatte beinhaltet.

28. Schützende Bekleidung gemäß Anspruch 1, wobei der schützende Bereich unter der Höhe der Hüftpfanne des Beckens auf der Seite des Körpers ungefähr zentriert ist.

29. Schützende Bekleidung gemäß Anspruch 1 oder Anspruch 28, wobei der schützende Bereich über der Hüftpfanne des Beckens auf der Vorderseite des Körpers ungefähr zentriert ist.

30. Schützende Bekleidung gemäß Anspruch 1, die ferner Mittel zum Anbringen des schützenden Polsters an einer von einer Vielzahl von unterschiedlichen Stellen innerhalb der schützenden Bekleidung beinhaltet.

## Revendications

1. Un vêtement de protection destiné à protéger l'énarthrose d'une hanche d'une personne qui le porte contre un choc, comportant :
une zone protectrice comportant un coussinet amortisseur de choc ;
une zone non protectrice ;
le coussinet de protection étant dimensionné et positionné pour couvrir l'avant, l'arrière et le côté de l'énarthrose lorsque le vêtement est porté par la personne qui le porte ;
**caractérisé en ce que** le coussinet de protection est muni d'une pluralité de fentes d'intersection.

2. Le vêtement de protection de la revendication 1, dans lequel ledit coussinet protecteur est résilient.

3. Le vêtement de protection de la revendication 1, dans lequel ledit coussinet de protection comporte un matériau en mousse.

4. Le vêtement de protection de la revendication 1, dans lequel ledit coussinet de protection est biseauté.

5. Le vêtement de protection de la revendication 3, dans lequel ledit matériau en mousse comporte un matériau en mousse à cellules fermées.

6. Le vêtement de protection de la revendication 3, dans lequel ledit matériau en mousse comporte un matériau en mousse à cellules ouvertes.

7. Le vêtement à coussinet de protection de la revendication 1, dans lequel ledit coussinet de protection comporte un coeur de coussinet et une enveloppe de coussinet.

8. Le vêtement de protection de la revendication 7, dans lequel ledit coeur de coussinet comporte plus d'une couche.

9. Le vêtement de protection de la revendication 8, dans lequel lesdites couches comportent des couches de différentes densités.

10. Le vêtement de protection de la revendication 7, dans lequel ladite enveloppe de coussinet entoure ledit coeur de coussinet.

11. Le vêtement de protection de la revendication 7, dans lequel ladite enveloppe de coussinet est formée de coton.

12. Le vêtement de protection de la revendication 7, dans lequel ladite enveloppe de coussinet comporte une étoffe d'interface en tricot de coton.

13. Le vêtement de protection de la revendication 1, dans lequel ledit coussinet de protection a une épaisseur d'entre 3 et 25 mm.

14. Le vêtement de protection de la revendication 1, dans lequel ladite zone non protectrice est formée d'étoffe.

15. Le vêtement de protection de la revendication 14, dans lequel ladite étoffe comporte du coton.

16. Le vêtement de protection de la revendication 14, dans lequel le matériau formant ladite étoffe est sélectionné dans le groupe consistant en nylon, lycra, polyester et spandex.

17. Le vêtement de protection de la revendication 1, dans lequel ledit coussinet de protection est assujetti de façon amovible à ladite zone non protectrice.

18. Le vêtement de protection de la revendication 1, dans lequel ledit coussinet de protection est fixé à ladite zone non protectrice.

19. Le vêtement de protection de la revendication 1, comportant une pluralité de coussinets de protection.

20. Le vêtement de protection de la revendication 17, dans lequel chaque coussinet de protection de ladite pluralité de coussinets de protection est disposé de façon adjacente à une région d'énarthrose.

21. Le vêtement de protection de la revendication 1, dans lequel ledit vêtement de protection comporte une gaine-culotte.

22. Le vêtement de protection de la revendication 1, comportant de plus un soutien d'abdomen.

23. Le vêtement de protection de la revendication 22, dans lequel ledit soutien d'abdomen est disposé sur le centre de l'avant dudit vêtement de protection.

24. Le vêtement de protection de la revendication 22, dans lequel ledit soutien d'abdomen comporte une interface non tissée.

25. Le vêtement de protection de la revendication 22, dans lequel ledit soutien abdominal comporte un matériau extensible.

26. Le vêtement de protection de la revendication 1, comportant de plus un soutien glutéal.

27. Le vêtement de protection de la revendication 1, comportant de plus un panneau de soutien du dos.

28. Le vêtement de protection de la revendication 1, dans lequel ladite zone protectrice est approximativement centrée sous le niveau dudit acétabulum dudit bassin sur ledit côté dudit corps.

29. Le vêtement de protection de la revendication 1 ou la revendication 28, dans lequel ladite zone protectrice est approximativement centrée sur ledit acétabulum dudit bassin sur l'avant dudit corps.

30. Le vêtement de protection de la revendication 1, comportant de plus un moyen destiné à attacher ledit coussinet de protection à un emplacement d'une pluralité d'emplacements différents au sein dudit vêtement de protection.
